Europäisches Patentamt

European Patent Office

Office européen des brevets

(11) Veröffentlichungsnummer: **0 292 757**
**A1**

## (12) EUROPÄISCHE PATENTANMELDUNG

(21) Anmeldenummer: 88107296.1

(22) Anmeldetag: 06.05.88

(51) Int. Cl.⁴: **A61K 7/30**

(30) Priorität: 27.05.87 DE 3717920

(43) Veröffentlichungstag der Anmeldung:
30.11.88 Patentblatt 88/48

(84) Benannte Vertragsstaaten:
**AT BE CH DE ES FR GB GR IT LI LU NL SE**

(71) Anmelder: **QUEISSER PHARMA GMBH & CO.**
**Schleswiger Strasse 74**
**D-2390 Flensburg(DE)**

(72) Erfinder: **Noronha, Rudolf V., Dr.**
**Falkenweg 4a**
**D-5024 Pulheim(DE)**

(74) Vertreter: **Patentanwaltsbüro Cohausz &**
**Florack**
**Schumannstrasse 97**
**D-4000 Düsseldorf 1(DE)**

(54) **Reinigungstablette für Zahnprothesen.**

(57) Die vorliegende Erfindung betrifft eine Pankreatin-Enzym enthaltende, lagerfähige Brausetablette für die Reinigung von Zahnprothesen.

EP 0 292 757 A1

## Reinigungstablette für Zahnprothesen

Die vorliegende Erfindung betrifft eine Pankreatin-Enzym enthaltende, lagerfähige Brausetablette für die Reinigung von Zahnprothesen.

Die Verschmutzungen einer Zahnprothese können in drei Gruppen eingeteilt werden, und zwar:

1) Speisereste fettigen Charakters oder fettig-viskosen Charakters (Mehlspeisen, Soßen, Fruchteis),
2) angelagerter Eiweißstoffe (Fleisch, Milchproduktreste) und
3) schwerwasserlösliche mineralische Ablagerungen von Speichel (zahnsteinähnlichen Charakters).

Die meisten Reinigungsmittel für Zahnprothesen sind in Form von Brausetabletten ausgebildet, die zusammen mit der Zahnprothese in Wasser gegeben werden und die neben Gasbildnern (z.B. Natriumhydrogencarbonat und Zitronensäure) verschiedene Salze und Produkte enthalten, die auf die vorgenannten Gruppen von Verunreinigungen ansprechen. Die in den meisten Tabletten vorhandenen Tenside und sauerstoffabspaltenden Substanzen sind als reinigendes Prinzip jedoch nicht voll befriedigend, insbesondere bei stark verschmutzten Prothesen. Selbst die Zuhilfenahme mechanischer Mittel kann die Zahnprothese häufig nicht voll reinigen derart, daß die Ursache für Geruchsbildung durch in der Prothese verbleibende geringfügige Speisereste und deren Verwesung vermieden wird.

Ansich würden sich die Speisereste oder einen Teil hiervon angreifende Enzyme zum Einbau in die Selbstreinigungsbrausetabletten für Zahnprothese anbieten. So sind in der EP-OS 28005 als übliche Zusätze geringe Mengen proteolytischer Enzyme als bekannt erwähnt. Oder in der kurze Zeit später angemeldeten DE-OS 3236966 wurde, allerdings in Verbindung mit Ultraschallbädern, d.h. mechanischen Mitteln, vorgeschlage, in eine Tablette aus einer leicht wasserlösliche, festen sauren Verbindung und einem leicht wasserlöslichen Tensid, ein Enzym, insbesondere Pepsin oder Papain einzubauen. Auf den Markt ist ein solches Produkt nie gelangt, wohl weil bei den aggressiven Bestandteilen der Tablette wie Zitronensäure Enzyme nicht dauerhaft beständig sind. Schließlich wurde in der DE-OS 3303330 vorgeschlagen, in die Brausreinigungstabletten das spezielle Enzym $\beta$-1,3-Glucanase einzubauen. Dieser Lösungsvorschlag ist jedoch nicht befriedigend, denn zum einen ist das spezielle Enzym nicht leicht erhältlich und zum anderen wird keine volle Reinigung der behandelten Zahnprothesen erreicht.

Aufgabe der vorliegenden Erfindung ist es, ein Produkt in Form üblicher wasserlöslicher Brausetabletten zu schaffen, mit deren Hilfe, ohne Einsatz zusätzlicher mechanischer Mittel und somit auf einfache Weise, Zahnprothesen von anhaftenden Speisen befreit werden können und diese Eigenschaft auch nach längerem Lagern erhalten ist.

Diese Aufgabe wird dadurch gelöst, daß zu einem Gemisch aus üblichen Produkten zur Erzeugung der Brausewirkung bei Zugabe der Tablette zu Wasser, üblichen Mitteln zur Entfernung anorganischer Ablagerungen wie Zahnstein und üblichen Zusätzen zur Geschmacks- und/oder Geruchsverbesserung das Enzym Pankreatin in Gegenwart eines Trockenmittels, insbesondere eines anorganischen Trockenmittels zugemischt wird. So enthält die erfindungsgemäße Tablette 10 bis 20 Gew.-%, vorzugsweise 10 Gew.%, Pankreatin zusammen mit 5 bis 15, vorzugsweise 10 Gew.% eines anorganischen Trockenmittels, bezogen auf die anderen Komponenten der Tablette. Als Trockenmittel kommen z.B. und insbesondere die wasserfreien Salze $CaCl_2$, $Na_2SO_4$ oder $Na_2CO_3$, insbesondere $Na_2SO_4$ infrage. $CaCl_2$ verbessert zusätzlich die enzymatische Aktivität und Stabilität.

Ausführungsbeispiel:

| Enzymatischer Reiniger | Versuch I | Versuch II |
|---|---|---|
| Pankreatin | 12,5 g | 12,5 g |
| $NaHCO_3$ | 32,5 g | 32,5 g |
| Citronensäure | 10,0 g | 10,0 g |
| Polyglykol 4000 | 5,0 g | 5,0 g |
| $Na_2SO_4$ | 12,0 g | 10,0 g |
| Texapon k 12 (L100) | 2,0 g | 2,0 g |
| Na-Polyphosphat | 15,0 g | 15,0 g |
| Na-Pyrophosphat | 6,0 g | 6,0 g |
| Silcron G - 910 | 1,0 g | 1,0 g |
| Optammit Öl | 0,2 g | 0,2 g |
| Farbstoff Poncean 4 R (Sicomet Erythrosin) E 124 | 0,035 g | 0,04 g |
| Amidosulfonsäure | – | 3,0 g |
| Benzoesäure | – | 3,0 g |

Die Komponenten werden sorgfältig gemischt und zu einer Tablette verpreßt.

## Ansprüche

1. Reinigungsbrausetablette für Zahnprothesen enthaltend eine in wässrigem Medium sauer reagierende Komponente und eine alkalisch reagierende Komponente als Bestandteil der Brausemischung und gegebenenfalls weiterer Zusätze, wie Aromastoffe, Farbstoffe, Indikatoren, Trägermittel und Trennmittel, **dadurch gekennzeichnet,** daß die Brausetablette 10 bis 20 Gew.-% Pankreatin in Kombination mit 5 bis 15 Gew.-% eines anorganischen Trockenmittels enthält.

2. Reinigungsbausetablette gemäß Anspruch 1, **dadurch gekennzeichnet,** daß sie 15 Gew.-% Pankreatin zusammen mit 10 Gew.-% des anorganischen Trockenmittels enthält.

3. Reinigungsbrausetablette gemäß Anspruch 1 oder 2, **dadurch gekennzeichnet,** daß das anorganische Trockenmittel ein wasserfreies Salz aus der Gruppe $CaCl_2$, $Na_2SO_4$ und $Na_2CO_3$ ist.

| | Europäisches Patentamt | **EUROPÄISCHER RECHERCHENBERICHT** | Nummer der Anmeldung |
| | | | EP 88 10 7296 |

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| Y | US-A-4 096 870 (J.A. MANFUSO)<br>* Ansprüche *<br>--- | 1-3 | A 61 K 7/30 |
| Y | DE-A-1 913 869 (COLGATE-PALMOLIVE)<br>* Ansprüche *<br>--- | 1-3 | |
| Y | DE-A-1 467 951 (IPTOR)<br>* Ansprüche 1,2 *<br>--- | 1-3 | |
| Y | GB-A-1 391 318 (MILES LABORATORIES)<br>* Ansprüche 1-3 *<br>--- | 1-3 | |
| Y | EP-A-0 028 005 (GERGELY)<br>* Anspruch 1 *<br>--- | 1-3 | |
| .Y | US-A-3 855 142 (PADER)<br>* Ansprüche 1-5 *<br>--- | 1-3 | |
| A,D | FR-A-2 520 614 (ROHTO)<br>* Ansprüche 1-3 *<br>----- | 1-3 | |
| | | | **RECHERCHIERTE SACHGEBIETE (Int. Cl.4)**<br><br>A 61 K 7/00<br>C 11 D 3/00 |

Der vorliegende Recherchenbericht wurde für alle Patentansprüche erstellt

| Recherchenort | Abschlußdatum der Recherche | Prüfer |
|---|---|---|
| BERLIN | 25-07-1988 | AVEDIKIAN P.F. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

······································································

& : Mitglied der gleichen Patentfamilie, übereinstimmendes Dokument

EPO FORM 1503 03.82 (P0403)